# EUROPEAN PATENT SPECIFICATION

(11) **EP 4 327 092 B1**
(45) Date of publication and mention of the grant of the patent: **30.09.2026**
(21) Application number: 22720886.5
(22) Date of filing: 18.04.2022
(51) Int. Cl.: G01N 21/77, G01N 21/78, G01N 21/64, G01N 31/22, G01N 33/18

(54) **MERCURY MEASUREMENT**
QUECKSILBER MESSUNG
MESURE DE MERCURE

(30) Priority: 19.04.2021 US 202117233942
(43) Date of publication of application: 28.02.2024
(73) Proprietor: Hach Company, Loveland, CO 80538-8842 (US)
(72) Inventor: DAS, Amit, Fort Collins, CO 80525 (US)
(74) Representative: Sandri, Sandro
(86) International application number: PCT/US2022/025204
(87) International publication number: WO 2022/225843

(56) References cited:
- CN-A- 1 752 750
- CN-A- 106 841 132
- CN-A- 108 084 133
- US-A1- 2020 386 682
- PAL SUMAN ET AL: "A highly selective chemodosimeter for fast detection and intracellular imaging of Hg 2+ ions based on a dithiocarbamate-isothiocyanate conversion in aqueous ethanol", ORGANIC & BIOMOLECULAR CHEMISTRY, vol. 12, no. 7, 1 January 2014 (2014-01-01), pages 1072 - 1078, XP055938939, ISSN: 1477-0520, DOI: 10.1039/C3OB42108B
- KO SUNG-KYUN ET AL: "In Vivo Monitoring of Mercury Ions Using a Rhodamine-Based Molecular Probe", JOURNAL OF THE AMERICAN CHEMICAL SOCIETY, vol. 128, no. 43, 6 October 2006 (2006-10-06), pages 14150 - 14155, XP055938937, ISSN: 0002-7863, DOI: 10.1021/ja065114a

## Description

### BACKGROUND

This application relates generally to measuring mercury in aqueous or liquid samples, and, more particularly, to the measurement of mercury using a thiocarbamate-based indicator.

Ensuring water quality is critical in a number of industries such as pharmaceuticals and other manufacturing fields. Additionally, ensuring water quality is critical to the health and well-being of humans, animals, and plants which are reliant on the water for survival. One element that is typically measured is mercury. Too much free mercury in water can be harmful to humans or animals, and regulated to be maintained below a regulated or desired level. Therefore, detecting the presence and concentration of mercury in water or other liquid solutions is vital.

Document CN 108084133 A, describes a rapid mercurial ion fluorescent probe with high selectivity and high sensitivity. A method for detecting mercury in a solution is described, wherein a thiocarbamate derivatized coumarin is used as the fluorescent reagent.

### BRIEF SUMMARY

The present invention relates to a method for measuring mercury in a solution, comprising the steps described at claim 1. The dependent claims outline advantageous ways of carrying out the method.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates a flow diagram of an example mercury measuring system.
FIG. 2 illustrates a chemical equation of an example thiocarbamate-based indicator for detection of mercury.
FIG. 3 illustrates an example emission spectral change for example mercury concentrations using a thiocarbamate-based indicator.
FIG. 4 illustrates an example fluorescence intensity measurement using a thiocarbamate-based indicator.
FIG. 5 illustrates an example of computer circuitry.

### DETAILED DESCRIPTION

Conventional methods of mercury measurement in water may have some limitations. For example, mercury measurement may be used to determine the quality of water. High concentrations of mercury may be harmful to animals, humans, and/or plants. Accordingly, as another example, a user or entity may want the mercury in a body of water to be under a particular threshold, therefore, the user may measure the mercury in order to determine if the amount of mercury is under that threshold.

Current methods, systems and kits for mercury measurement are cumbersome and require many steps. Multiple steps introduce errors into a mercury measurement. Additionally, mercury measurement requires a preconcentration step. Conventional methods are not accurate or detect or measure mercury in very low ranges. What is needed is a method to measure mercury without multiple steps and/or preconcentration of a sample.

Accordingly, the present invention relates to a method for measuring mercury at ultralow range (ULR) concentrations with improved reagent stability under ambient conditions. The amount of mercury in the water may be less than 200 parts per billion (ppb), 50 ppb, 20 ppb, 5 ppb, 2 ppb, or 1 ppb. Thus, the method detects mercury in concentrations below 200 ppb, and yields accurate concentration measurement of mercury as low or lower than 50 ppb, 20 ppb, 10 ppb, 5 ppb, 2 ppb, or 1 ppb. The method utilizes a fluorometric method. The indicator to give a fluorescence signal is a thiocarbamate derivative. The thiocarbamate derivative is a coumarin based fluorophore. According to the present invention the thiocarbamate-based indicator is a umbelliferone or 4-methylumbelliferone thiocarbamate. The fluorescence intensity is correlated to measurement and detection of mercury. The pH of a solution is adjusted to a pH around 6 to activate the reporter or indicator molecule.

Furthermore, a system is provided for measuring mercury at ultralow range (ULR) concentrations. The system is not part of the present invention.

The illustrated example embodiments will be best understood by reference to the figures.

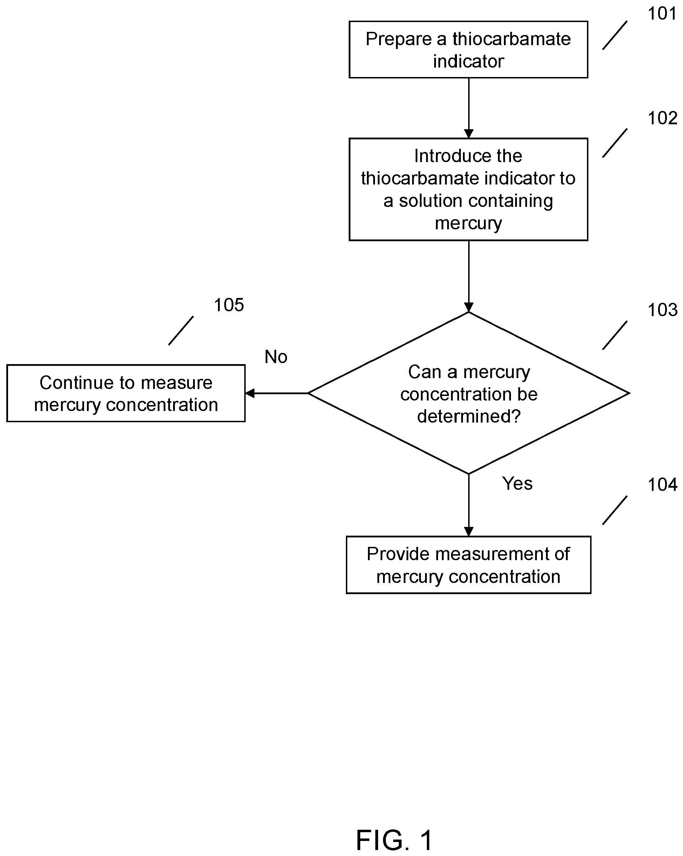

Referring to FIG. 1, a method for detection of mercury in solution is illustrated. A thiocarbamate-based indicator is prepared. The thiocarbamate-based indicator is introduced to a solution containing mercury. The thiocarbamate-based indicator in the presence of mercury causes a change in fluorescence intensity of the thiocarbamate-based indicator. The change of fluorescence intensity is correlated to a concentration of mercury in the solution.

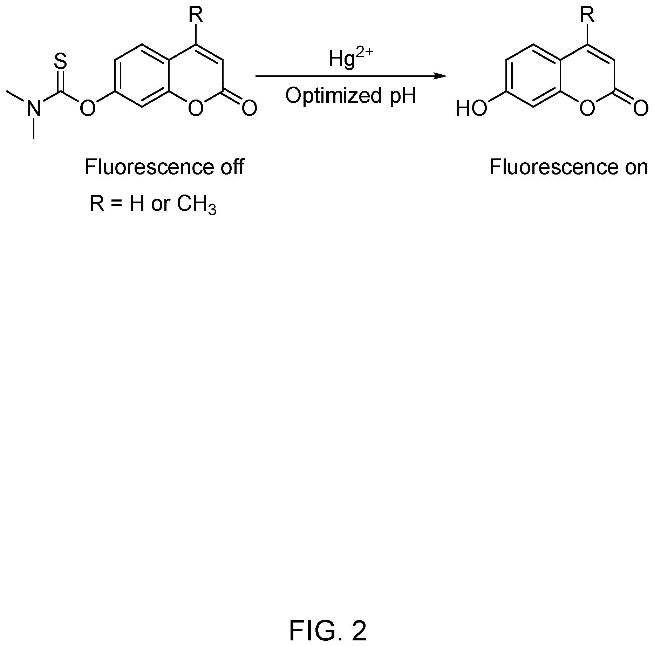

At 101, a thiocarbamate-based indicator is prepared. The thiocarbamate is a thiocarbamate derivative of a coumarin based fluorophore. The thiocarbamate-based indicator may be umbelliferone or 4-methylumbelliferone thiocarbamate. Referring to FIG. 2, an example reaction of the thiocarbamate-based indicator is illustrated. The thiocarbamate-based indicator detects mercury in the ultralow range of less than 200 ppb. The thiocarbamate-based indicator remains stable for long periods of storage. For example, storage of 4 months does not diminish the accurate determination of mercury in solution.

At 102, the thiocarbamate-based indicator is introduced into a solution. The solution contains mercury or an amount of mercury. The solution is an aqueous sample which may include a sample from a natural body of water, a holding tank, a processing tank, a pipe, or the like. The solution may be in a continuous flow, a standing volume of liquid, or any combination thereof. The solution is introduced to the thiocarbamate-based indicator, for example, a test chamber of the measurement device. The measurement device may be a hand held device. A hand held device has advantages such as lower cost, portability, field use, or the like. Alternatively, the measurement device may be a larger bench top device. Introduction of the solution into the measurement device includes placing or introducing the solution into a test chamber manually by a user or using a mechanical means, for example, gravity flow, a pump, pressure, fluid flow, or the like. For example, a water sample for mercury testing is introduced to a measurement or test chamber using a pump. Valves or the like control the influx and efflux of the solution into or out of the one or more chambers, if present.

Additionally or alternatively, the measurement device may be present or introduced in a volume of the solution. The measurement device is then exposed to the volume of solution where it performs measurements. The system may be a flow-through system in which a solution and/or reagents are automatically mixed and measured. Once the sample is in contact with the measurement system, the system measures the mercury or an amount of mercury of the sample, as discussed in further detail herein. The measurement device may include one or more chambers in which the one or more method steps are performed.

Measurement of mercury in water or an aqueous sample utilizes a thiocarbamate-based indicator. The thiocarbamate-based indicator comprises umbelliferone and/or 4-methylumbelliferone. Mercury in +2 oxidation state has strong affinity for sulfur and binds efficiently with sulfur containing compounds. Hg²⁺ rips the sulfur off from the molecule and produce mercuric sulfide (HgS).

7-hydroxy coumarin and its substituted derivatives are highly fluorescent. When the hydroxy group is being protected using N,N-dimethylthiocarbamoyl group, the molecule becomes very weak fluorescent to nonfluorescent. Upon reaction of the indicator with Hg²⁺ in water, results in the formation of the reporter molecule with increase in fluorescence intensity. (*See* FIG. 2).

The pH of the solution is maintained at a pH of between 5 and 7. Advantageously, the pH of the solution is maintained at a pH of approximately 6.0. For example, the pH is adjusted or titrated to around a pH of 6.0. The concentration of thiocarbamate in the thiocarbamate-based indicator is between 1 and 10 µM, preferably between 4 - 6 µM. A phosphate buffer is added. Increasing the amount of buffer decreases fluorescence intensity. Saline may be added to the solution. The saline does not affect the fluorescence intensity.

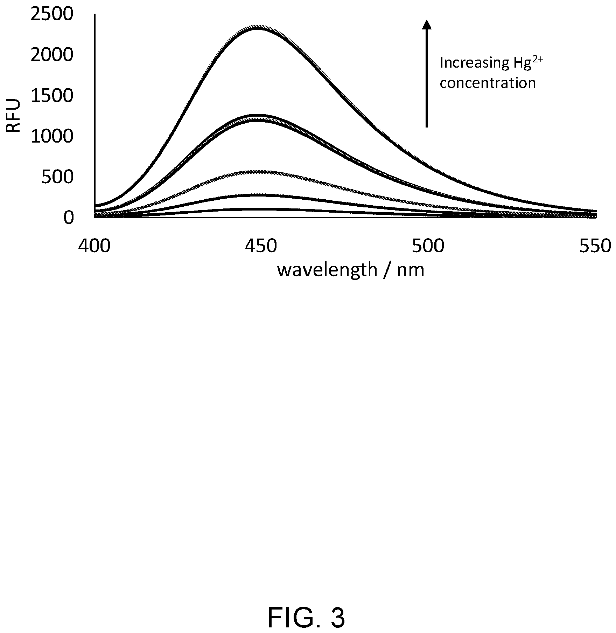

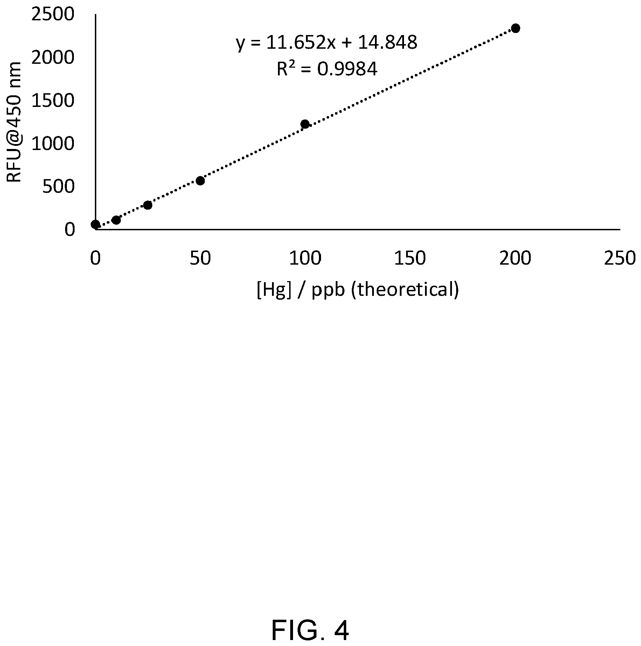

At 103, the method determines if a mercury concentration or an amount of mercury is measured. The presence of mercury in an aqueous solution causes an increase in fluorescence intensity of the thiocarbamate-based indicator. The thiocarbamate derivative is selective for mercury and reacts with mercury in an aqueous environment releasing a fluorescence active molecule. Examples of this increase in fluorescence intensity and dose response curves for a thiocarbamate-based indicator are illustrated in FIG. 3 and FIG. 4. Therefore, the fluorescence intensity, of a solution containing mercury is correlated to the concentration of the mercury in the aqueous solution. Fluorescence curves may be generated for a range of mercury concentrations, for different thiocarbamate-based indicators, for any different condition that may affect absorption or fluorescence values (e.g., temperature, sample content, turbidity, viscosity, measurement apparatus, aqueous sample chamber, etc.), or the like.

An approximate range of detection of mercury is between 0 - 200 ppb. Referring to FIG. 3, an emission spectral change by varying mercury concentration or amount in water is illustrated. The reaction mixture was excited at 325 nm wavelength and emission was monitored from 400 nm to 550 nm. FIG. 3 shows the emission spectral change when concentration of Hg²⁺ was varied from 0 ppb to 200 ppb. An increase in fluorescence intensity was observed on increasing concentration of Hg²⁺ in water.

Referring to FIG. 4, a calibration plot for mercury detection in water is illustrated. A dose response curve or calibration plot is generated by plotting emission intensity at 450 nm wavelength (λex = 325 nm) versus the theoretical concentration of Hg²⁺. A linear response was observed for 0 - 200 ppb concentration range of mercury.

Mercury concentration measurement may be at periodic intervals set by the user or preprogrammed frequencies in the device. Measurement of mercury by a device allows for real time data with very little human involvement in the measurement process. Cleaning of the fluorometric chamber may be required at an unspecified time interval. A programmed calibration curve may be entered into the device.

A chamber, vessel, cell, chamber, or the like may contain an aqueous sample, at least one thiocarbamate-based indicator, and associated reagents such as buffers and/or additives. A device may contain one or more bottles of reagents which contain necessary reagents. The reagents contained in the one or more bottles may be pump fed or gravity fed. The flow of the reagents may be metered to ensure proper volume delivery to the measurement cell. The aqueous sample may be fed through a pressured inlet, a vessel, or the like. The aqueous sample may be introduced into the measurement chamber by a pump or gravity fed. The sampling device may be in series or parallel to an aqueous flow. The device may have a system to ensure proper mixing of the aqueous sample, thiocarbamate-based indicator, and related reagents.

The fluorescence intensity or mercury concentration may be an output upon a device in the form of a display, printing, storage, audio, haptic feedback, or the like. Alternatively or additionally, the output may be sent to another device through wired, wireless, fiber optic, Bluetooth^{®}, near field communication, or the like. An alarm may be used to warn of a measurement or concentration outside acceptable levels. A system may be used to shut down water output or shunt water from sources with unacceptable levels of an analyte. For example, an analyte measuring device may use a relay coupled to an electrically actuated valve, or the like.

At 105, if a concentration of mercury cannot be determined, the system continues to measure mercury. Additionally or alternatively, the system may output an alarm, log an event, or the like.

At 104, if a concentration or amount of mercury can be determined, the method provides a measurement of mercury concentration. The system may connect to a communication network. The system may alert a user or a network. This alert may occur whether a mercury measurement is determined or not. An alert may be in a form of audio, visual, data, storing the data to a memory device, sending the output through a connected or wireless system, printing the output or the like. The system may log information such as the measurement location, a corrective action, geographical location, time, date, number of measurement cycles, or the like. The alert or log may be automated, meaning the system may automatically output whether a correction was required or not. The system may also have associated alarms, limits, or predetermined thresholds. For example, if a mercury concentration reaches a threshold. Alarms or logs may be analyzed in real-time, stored for later use, or any combination thereof.

The various embodiments described herein thus represent a technical improvement to conventional mercury measurement techniques. Using the techniques as described herein, a thiocarbamate-based indicator is used to measure mercury in solution. Such techniques provide a faster and more accurate method for measuring mercury in an aqueous or water sample.

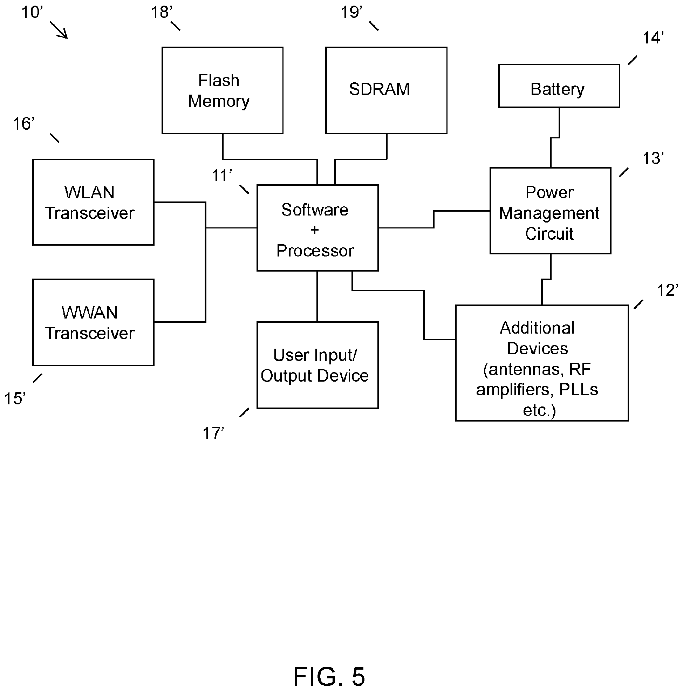

While various other circuits, circuitry or components may be utilized in information handling devices, with regard to an instrument for measurement of mercury according to any one of the various embodiments described herein, an example is illustrated in FIG. 5. Device circuitry 10' may include a measurement system on a chip design found, for example, a particular computing platform (e.g., mobile computing, desktop computing, etc.) Software and processor(s) are combined in a single chip 11'. Processors comprise internal arithmetic units, registers, cache memory, busses, I/O ports, etc., as is well known in the art. Internal busses and the like depend on different vendors, but essentially all the peripheral devices (12') may attach to a single chip 11'. The circuitry 10' combines the processor, memory control, and I/O controller hub all into a single chip 11'. Also, systems 10' of this type do not typically use SATA or PCI or LPC. Common interfaces, for example, include SDIO and I2C.

There are power management chip(s) 13', e.g., a battery management unit, BMU, which manage power as supplied, for example, via a rechargeable battery 14', which may be recharged by a connection to a power source (not shown). In at least one design, a single chip, such as 11', is used to supply BIOS like functionality and DRAM memory.

System 10' typically includes one or more of a WWAN transceiver 15' and a WLAN transceiver 16' for connecting to various networks, such as telecommunications networks and wireless Internet devices, e.g., access points. Additionally, devices 12' are commonly included, e.g., a transmit and receive antenna, oscillators, PLLs, etc. System 10' includes input/output devices 17' for data input and display/rendering (e.g., a computing location located away from the single beam system that is easily accessible by a user). System 10' also typically includes various memory devices, for example flash memory 18' and SDRAM 19'.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an embodiment to perform measurement of mercury of an aqueous sample.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and products according to various example embodiments. It will be understood that the actions and functionality may be implemented at least in part by program instructions. These program instructions may be provided to a processor of a device, e.g., a hand held measurement device, or other programmable data processing device to produce a machine, such that the instructions, which execute via a processor of the device, implement the functions/acts specified.

It is noted that the values provided herein are to be construed to include equivalent values as indicated by use of the term "about." The equivalent values will be evident to those having ordinary skill in the art, but at the least include values obtained by ordinary rounding of the last significant digit.

System 10' typically includes one or more of a WWAN transceiver 15' and a WLAN transceiver 16' for connecting to various networks, such as telecommunications networks and wireless Internet devices, e.g., access points. Additionally, devices 12' are commonly included, e.g., a transmit and receive antenna, oscillators, PLLs, etc. System 10' includes input/output devices 17' for data input and display/rendering (e.g., a computing location located away from the single beam system that is easily accessible by a user). System 10' also typically includes various memory devices, for example flash memory 18' and SDRAM 19'.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an embodiment to perform measurement of mercury of an aqueous sample.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and products according to various example embodiments. It will be understood that the actions and functionality may be implemented at least in part by program instructions. These program instructions may be provided to a processor of a device, e.g., a hand held measurement device, or other programmable data processing device to produce a machine, such that the instructions, which execute via a processor of the device, implement the functions/acts specified.

## Claims

1. A method for detecting and measuring mercury at concentrations lower than 200 parts per billion (ppb) in a an aqueous solution containing mercury, comprising:
preparing (101) a thiocarbamate-based indicator comprising umbelliferone and/or 4-methylumbelliferone at a concentration between 1 and 10 µM, as well as a phosphate buffer;
introducing (102) the thiocarbamate-based indicator to the solution, wherein the pH of the solution is adjusted to a value around 6.0 to activate the thiocarbamate-based indicator, whereby the introducing causes a change in fluorescence of the solution; and
measuring (104) the concentration of mercury in the solution which is correlated to the intensity of the fluorescence.

2. The method of claim 1, wherein the solution comprises a water sample.

3. The method of claim 1, wherein the amount of mercury removes the sulfur from the thiocarbamate-based indicator increasing fluorescence intensity of the thiocarbamate-based indicator.

4. The method of claim 1, wherein the measuring comprises measuring the mercury concentration with a hand-held device.

5. The method of claim 4, wherein the solution is introduced with the indicator into a test chamber of the hand-held device.

6. The method of claim 1, wherein fluorescence curves are generate of a range of mercury concentrations.

## Patentansprüche

1. Verfahren zum Nachweis und zur Messung von Quecksilber in Konzentrationen unter 200 Teile pro Milliarde (ppb) in einer quecksilberhaltigen wässrigen Lösung, umfassend:
Herstellen (101) eines Indikators auf Thiocarbamat-Basis, der Umbelliferon und/oder 4-Methylumbelliferon in einer Konzentration zwischen 1 und 10 µM sowie einen Phosphatpuffer enthält;
Zugabe (102) des Indikators auf Thiocarbamatbasis zu der Lösung, wobei der pH-Wert der Lösung auf einen Wert um 6,0 eingestellt wird, um den Indikator auf Thiocarbamatbasis zu aktivieren, wodurch die Zugabe eine Änderung der Fluoreszenz der Lösung bewirkt; und
Messung (104) der Quecksilberkonzentration in der Lösung, die mit der Intensität der Fluoreszenz korreliert.

2. Verfahren nach Anspruch 1, **dadurch gekennzeichnet, dass** die Lösung eine Wasserprobe umfasst.

3. Verfahren nach Anspruch 1, **dadurch gekennzeichnet, dass** die Quecksilbermenge den Schwefel aus dem Indikator auf Thiocarbamatbasis entfernt, wodurch die Fluoreszenzintensität des Indikators auf Thiocarbamatbasis erhöht wird.

4. Verfahren nach Anspruch 1, **dadurch gekennzeichnet, dass** das Messen, das Messen der Quecksilberkonzentration mit einem Handgerät umfasst.

5. Verfahren nach Anspruch 4, **dadurch gekennzeichnet dass** die Lösung zusammen mit dem Indikator in eine Testkammer des Handgeräts eingebracht wird.

6. Verfahren nach Anspruch 1, **dadurch gekennzeichnet, dass** Fluoreszenzkurven für einen Bereich von Quecksilberkonzentrationen erstellt werden.

## Revendications

1. Procédé pour détecter et mesurer le mercure à des concentrations inférieures à 200 parties par milliard (ppb) dans une solution aqueuse contenant du mercure, comprenant :
la préparation (101) d'un indicateur à base de thiocarbamate comprenant de l'umbelliférone et/ou de la 4-méthylumbelliférone à une concentration comprise entre 1 et 10 µM, ainsi qu'un tampon phosphate ;
l'introduction (102) de l'indicateur à base de thiocarbamate dans la solution, dans lequel le pH de la solution est ajusté à une valeur d'environ 6,0 pour activer l'indicateur à base de thiocarbamate, moyennant quoi l'introduction provoque une variation de la fluorescence de la solution ; et
la mesure (104) de la concentration de mercure dans la solution qui est corrélée à l'intensité de la fluorescence.

2. Procédé selon la revendication 1, dans lequel la solution comprend un échantillon d'eau.

3. Procédé selon la revendication 1, dans lequel la quantité de mercure retire le soufre de l'indicateur à base de thiocarbamate, augmentant l'intensité de fluorescence de l'indicateur à base de thiocarbamate.

4. Procédé selon la revendication 1, dans lequel la mesure comprend la mesure de la concentration de mercure avec un dispositif portatif.

5. Procédé selon la revendication 4, dans lequel la solution est introduite avec l'indicateur dans une chambre d'essai du dispositif portatif.

6. Procédé selon la revendication 1, dans lequel des courbes de fluorescence sont générées pour une gamme de concentrations de mercure.
